# EUROPEAN PATENT APPLICATION

(11) **EP 0 963 834 A1**
(43) Date of publication of application: **15.12.1999**
(21) Application number: 98304496.7
(22) Date of filing: 08.06.1998
(51) Int. Cl.: B29C 67/00, B22C 7/02, E04G 1/00, A61L 27/00, D04H 13/00, B01D 39/14

(54) **Scaffold material with a self-stabilizing structure**

(71) Applicant: Molecular Geodesics, Inc., Boston, Massachusetts 02199 (US)
(72) Inventor: Ingber, Donald E., Boston, Massachusetts 02116 (US)
(74) Representative: Greenwood, John David

(57) **Abstract**

A scaffold material is provided which comprises a predetermined arrangement of integrally connected modules, each said module comprised of a plurality of integrally connected elongated members forming at least a portion of a polyhedron, the members arranged such that at least a portion of said members form geodesic or tensegrity elements.

The scaffold material can be used in various ways, such as in filtration material, catalysis, protective textile fabrics, production of patterns for making moulds by shell investment casting, and biomedical applications, e.g. detoxification.

## Description

### Field of the Invention

This invention relates to three-dimensional structures which possess geodesic features and can stabilize through use of tensegrity.

### Background of the Invention

There is a need to develop new light-weight, porous materials that exhibit enhanced mechanical strength, flexibility and exposed surface as scaffolds for detoxification, filtration, catalysis, textile fabrics, space-filling, space-covering and biomedical applications. The ultimate material would be biomimetic materials that mimic the mechanical responsiveness and bioprocessing capacities of living cells and tissues.

Living cells and tissues use tensegrity architecture to organize and mechanically stabilize their internal filamentous support networks (interconnected nuclear matrix, cytoskeletal, and extracellular matrix scaffolds) and hence, their three dimensional forms (see, *J. Cell Sci*. 104:613,1993). The concept of tensegrity is well-known in the fabrication of geodesic structures, such as geodesic domes. See, for example, U.S.Patent Nos. 3,063,521, 3,354,591 and 4,901,483. Tensegrity construction is based upon the realization that most building materials are much more efficiently utilized and can often withstand higher forces when in tension than when in compression. In tensegrity construction, there is a high ratio of tension to compression elements. The tension members in these structures are geodesic elements that delineate the shortest distance between vertices that define an enclosed polyhedron. The mathematical modeling rules for the building and designing of tensegrity structures is also well understood. See, for example, Kenner in Geodesic Math (1980) in which the basic mathematics defining the orientation of individual structural elements within simple tensegrity modules is described. These models have been directed to macrostructures suitable for use in large-scale objects, such as buildings and toys. See, U.S. Patent No. 3,695,617. These macrostructures have been prepared by joining individual elements in the desired three dimensional arrangement and hence are not well suited to miniaturization.

Thus, there remains a need to prepare materials which can exhibit the mechanical responsiveness and bioprocessing capabilities of living cells and tissues. This invention is based on recent advances that have been made in our understanding of the relation between microstructure and macroscopic behavior in living cells and tissues.

### Summary of the Invention

It is an object of the present invention to provide a material having novel mechanical and bioactive properties. It is a further object of the invention to provide a material useful as a protective shield or textile and for filtration, detoxification and biomedical applications.

The present invention describes three-dimensional biomimetic scaffold materials which possess geodesic features, can stabilize through use of tensegrity and may be fabricated as unitary structures on the micro or macro scale.

In one aspect of the invention, a scaffold material is provided which possesses a predetermined arrangement of integrally connected modules, each said module comprised of a plurality of integrally connected elongated members forming at least a portion of a polyhedron, the members arranged such that at least a portion of said members form geodesic or tensegrity elements.

By "scaffold," as that term is used herein, it is meant a material having an extended repeating structure, which forms a framework or skeleton onto which and into which additional components may be introduced to impart additional features to the material.

By "module," as that term is used herein, it is meant a plurality of integrally connected structural members that delineate the edges of at least a portion of a polyhedron.

By "integrally connected," as that term is used herein, it is meant a single composition or structure made up of a plurality of elements to form a single unitary body. The structure does not posses discrete connectors or additional bonding or adhesive materials.

By "geodesic element," as that term is used herein, it is meant a geometric element which defines the shortest distance between two points on the surface of a solid. For example, a line is the shortest distance between two vertices on a surface of a polyhedron, a path along a great circle is the shortest distance (and hence, a geodesic element) for a sphere, and a spiral is a geodesic element on the surface of a cylinder. A triangle is geodesic because it represents the shortest, most economical path between three vertices on the surface of a polyhedron.

By "tensegrity element," as that term is used herein, it is meant an arrangement of interconnected structural members that self-stabilizes through transmission of continuous tension and discontinuous compression. Tensegrity elements may be composed of members that selectively resist tension or compression locally or of all non-compressible members that may resist either tension or compression depending on their location and the path of force transmission. A triangle composed of all non-compressible struts is an example of the latter type of self-stabilizing tensegrity structure.

By "extensible element," as that term is used herein, it is meant an element that is capable of extension or an increase in the length of the member within a given range of movement in response to application of a tensile force to one or both ends of the member.

By "non-compressible element," as that term is used herein, it is meant an element that is incapable of shortening along its length when compressive forces are applied to one or both ends of the member. However, the non-compressible member may be able to buckle under compression, without shortening its length. A non-compressible member may or may not be able to extend in length when external tensile forces are applied to its ends. Such an extensible, non-compressible member would be able to withstand compression, but not tension.

### Brief Description of the Drawing

The present invention is described with reference to the following Figures, which are presented for the purposes of illustration only and which are by no means intended to be limiting of the invention and in which:
Figure 1 is an illustration of a cuboctahedron module containing non-compressible elongated members which can rearrange to self-stabilize through tensegrity;
Figure 2(a) illustrates a hierarchical, nucleated tensegrity "cell" model composed of sticks and strings and Figure 2(b) illustrates the coordinated spreading of the cell and nucleus that occurs when living cells adhere to an adhesive substrate;
Figure 3 is a plot of mechanical stiffness (ratio of stress to strain) v. applied stress for living cells (Fig. 3a) and for a tensegrity model (Fig. 3b);
Figure 4 is an illustration of a fully geodesic scaffold material of the present invention using non-compressible elongated members and tetrahedron modules;
Figure 5 is an illustration of a flexible geodesic scaffold material composed of cuboctahedron modules impregnated with a swellable polymer before swelling (Fig. 5a) and after swelling localized hydrogel islands (Fig. 5b) so that the scaffold material is prestressed and stiffened while retaining open pores;
Figure 6 is a computer simulation illustrating a series of dynamic geometric transformations within an integrally connected scaffold composed of three cuboctahedron modules that are joined along a single edge, which is capable of rearranging from a linear, flexible cuboctahedral array containing a large common central pore to form a linear, rigid, closed octahedral tensegrity arrangement lacking any pore;
Figure 7 is a computer simulation illustrating a series of dynamic geometric transformations within three cuboctahedron modules that are joined along a different single edge than that shown in Fig. 6, which is capable of rearranging from a linear, flexible cuboctahedral array to form a nonlinear, rigid, closed octahedral tensegrity structure that exhibits a "dog-leg" geometry;
Figure 8 is a computer simulation illustrating a series of dynamic geometric transformations within three cuboctahedron modules that are joined along a common triangular face, which is capable of rearranging from a linear, flexible cuboctahedral array to form a nonlinear, rigid, closed octahedral tensegrity arrangement different than that shown for Fig. 6;
Figure 9 is an illustration of a scaffold material coated with a hydrogel (Fig. 9a) and impregnated with an additional detoxification agent (Fig. 9b);
Figure 10 is a computer simulation illustrating that the addition of larger fibers that are stiff, yet flexible, to a modular network results in the increased resistance to distortion and a tensegrity network that stiffens and is held open when stressed;
Figure 11 is an illustration of a geodesic material composed of all extensible members arranged into a spherical module;

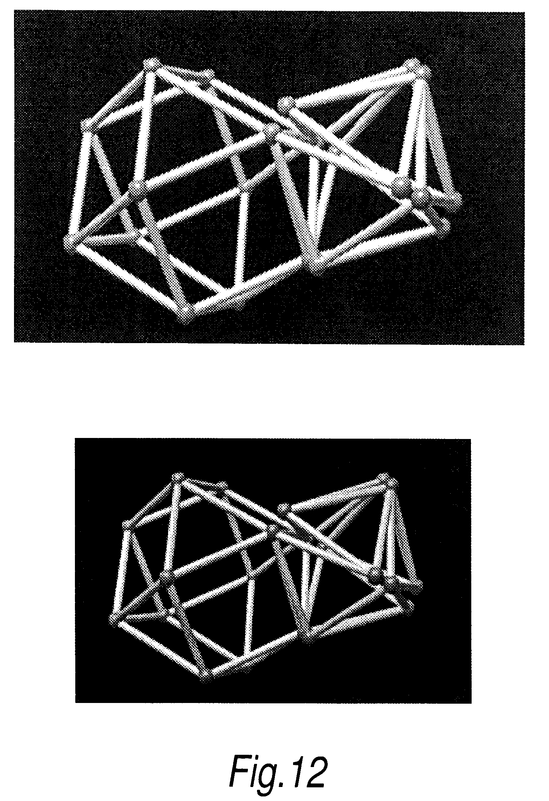

Figure 12 is an illustration of two cuboctahedron in different stages of contraction and rearrangement which demonstrates that the individual modules are not required to behave in a concerted fashion; and
Figure 13 is an illustration of a computer model of the deformation of two linked cuboctohedra (Fig. 13a and 13b), and of the corresponding deformation of two linked cuboctohedra fabricated by CAD/CAM methods (Fig. 13c and 13d).

### Detailed Description of the Invention

The present invention applies the rules of biological cell and tissue organization as well as novel features of cellular biochemistry to the design and fabrication of synthetic materials with mechanical, structural and chemical processing abilities similar to those of living tissues and cells.

The stability of most man-made structures requires that compressive forces, caused by the pull of gravity, be transmitted continuously across all key supporting elements, a stone arch being a simple example. In contrast, tensegrity structural stabilization occurs when the structural members are arranged geodesically such that only a subset of isolated struts bear compression and, instead, tension is continuous. In engineering terms, it describes a building system that self-stabilizes through inclusion of isolated compression struts that place surrounding structural elements under tension or that resist the inward-directed pull of surrounding contractile or shrinkable tensile networks and thereby impose a prestress in the entire structure. The simplest tensegrity unit therefore might be viewed as a tensile string pulling against a single incompressible linear strut, e.g., a bow and bowstring. The prestress would be the internal tension that is equilibrated in the system prior to force application. Tensegrity modules may be organized together using similar building rules to create polyhedral modules that self-stabilize through tensegrity and their geodesic arrangement. By interconnecting multiple individual geodesic modules using similar building rules, higher order hierarchical structures may be assembled.

In all tensegrity structures, a local applied stress results in long-range transfer of tensile forces. When all structural members are non-compressible and all are arranged geodesically, as in a geodesic dome or octet truss, the entire structure is mechanically stable and exhibits enhanced mechanical load-bearing capabilities.

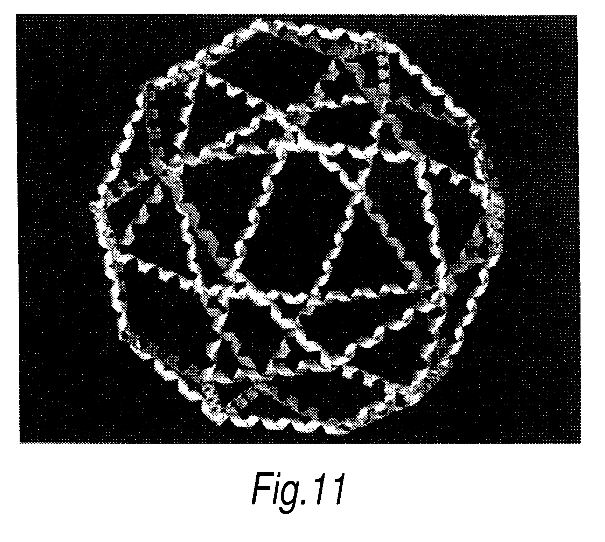

When structural members are extensible, the entire structure will distend while maintaining an overall shape (e.g., remaining a sphere at all levels of distension) and will geodesically rearrange in response to an applied stress. This structure will not self-stabilize except when the distending stress is released and allowed to contract and some or all of its extensible members have reached a non-compressible, compression-resistant configuration. An example of such a structure is shown in Fig. 11, in which the scaffold material is comprised of a plurality of geodesic integrally connected extensible modules. The structure will change size or degree of extension when external stress is applied while maintaining the pattern of intermember relationships constant. Multiple dome modules such as shown in Fig. 11 may be integrally connected by a planar arrangement of geodesic elements, e.g., triangles of similar structural members.

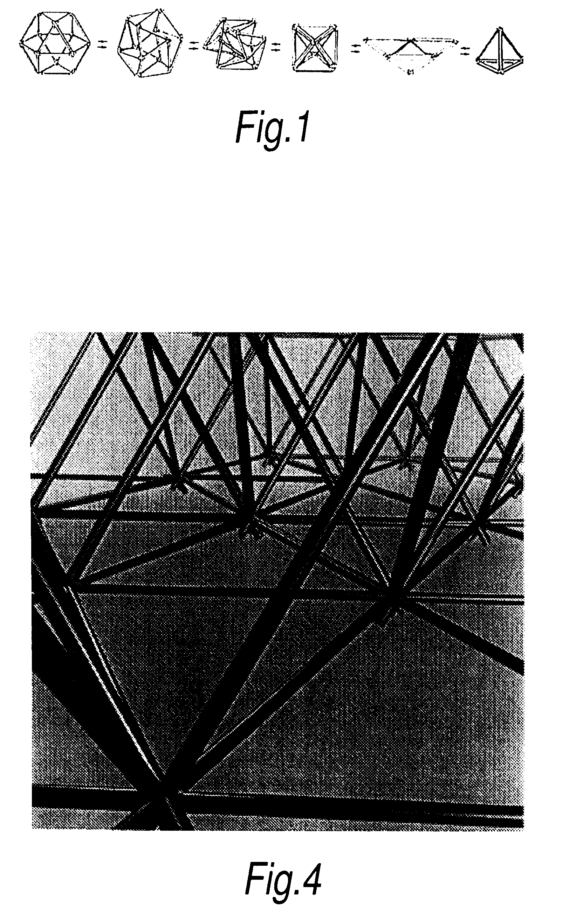

When all structural members are non-compressible and only a subset of members are arranged geodesically, the stable geodesic triangulated elements can kinematically rearrange in response to applied stress by changing the angle between members of adjacent geodesic elements at each vertex until all of the members reorganize to form a fully geodesic polyhedral module, such as an octahedron or tetrahedron. The entire structure then again becomes mechanically stable and exhibits high mechanical strength. Fig. 1 demonstrates the dynamic structural transformations possible in the three dimensional cuboctahedron module. The cuboctahedron module has four triangles around each square opening. The transformation between a flexible polyhedral network and stiff octahedral and tetrahedral forms results from inward pulling and twisting. This model predicts structural transformations that are observed in the actin skeleton of living cells at the cellular level and within the lung alveoli at the tissue level. See, D. Ingber *J. Cell Sci.* **104**:613 (1993).

When only the tensile members are extensible or when all members are non-extensible, but the compression struts are non-compressible or buckleable, a local applied stress will result in global structural rearrangements and a proportional or linear increase in the mechanical stiffness of the entire structure as the level of applied stress is raised (Wang *et al*, 1993 *Science*; Stamenovic *et al*., 1996 *J. Theor. Biol*; Fuller synergetics). This linear increase in stiffness in response to applied stress is also a fundamental property of living cells and tissues.

The novel material disclosed herein is based upon the discovery that architectural principles that govern the microstructure of biological elements play a role in the responsiveness of living cells and tissues. The present invention applies these architectural principles to the fabrication of synthetic materials which mimic the mechanical, structural and chemical properties of living tissue.

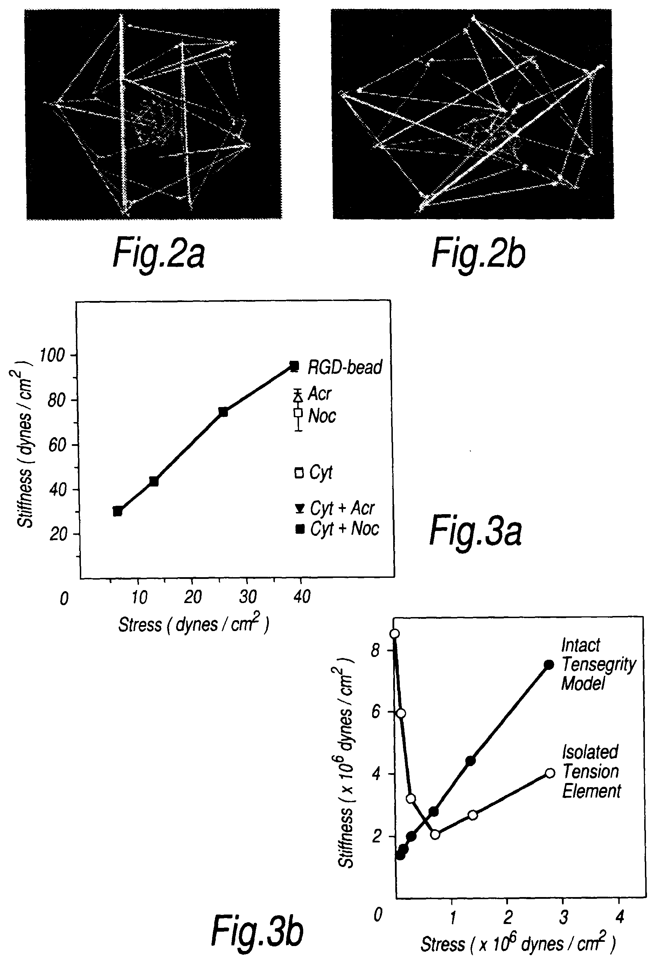

It has been demonstrated that living cells use tensegrity architecture to organize their cytoskeleton and to stabilize themselves against shape distortion. See, *J. Cell Sci. supra*, and *J. Theor Bio.* **181**:125 (1996), herein incorporated in their entirety by reference. This type of building system self-stabilizes by incorporating isolated compression elements (e.g., molecular struts, localized swelling pressures) that place the surrounding network under tension and thereby impose a prestress in the entire structure. These structures undergo global structural rearrangements and geometric transformations when external stress is applied locally (Figs. 2(a) and 2(b)). Tensegrity networks containing flexible joints also are able to undergo extensive geometric transformation even when the individual support struts are inflexible (Fig. 1). The most familiar examples of tensegrity architecture are the geodesic domes of Buckminster Fuller and the musculoskeletal framework of man. In fact, the enhanced load-bearing capabilities of all geodesic structures (domes, octet trusses, etc.) are due to a tensegrity-based mechanism of stress transmission and self-stabilization.

Another feature of tensegrity structures is that their mechanical stiffness increases in direct proportion as the level of applied stress is raised (Fig. 3b). This is a fundamental property of all living cells (Fig. 3a) and tissues, including human skin, and is responsible for their characteristic tensile strength and mechanical flexibility. In another feature of cellular structure, much of cellular metabolism functions in a solid state when immobilized on the insoluble cytoskeleton of the cell. Thus, mechanically-induced changes in cytoskeletal structure and mechanics will affect the behavior of biochemical processing molecules that associate with the cytoskeleton. Where the biologically-active species is load-bearing, such as cytoskeletal filaments and associated bioactive molecules including enzymes, then the mere change in cytoskeletal structure (and hence distortion of these molecules) may induce changes in chemical potential and thermodynamic states in the system. See, D. Ingber, "Tensegrity: The Architectural Basis of Cellular Mechanotransduction," *Annu. Rev. Physiol.* **59**: 575-599 (1997).

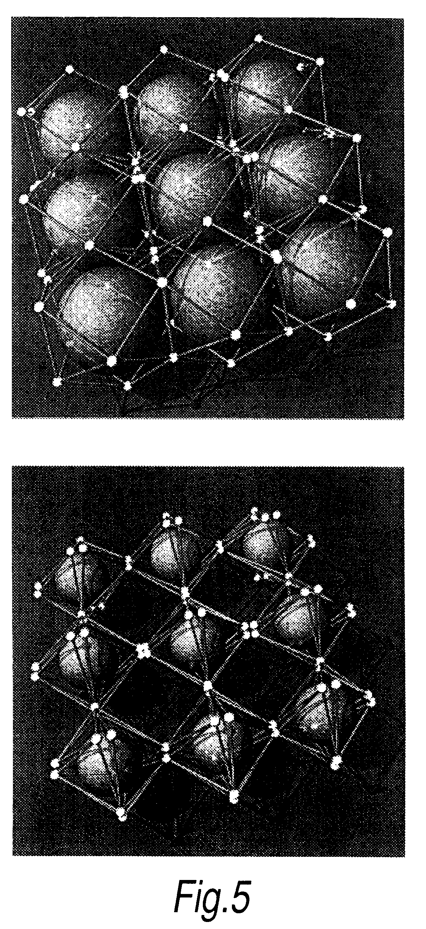

According to the present invention, the scaffold biomimetic material comprises a predetermined arrangement of integrally connected modules. Each module is comprised of a plurality of integrally connected elongated members which form at least a portion of a polyhedron. The elongated members are arranged such that at least a portion of the members form geodesic or tensegrity elements. Because the scaffold material encompasses geodesic and/or tensegrity elements, the material exhibits superior mechanical strength and responsiveness. A typical scaffold material is shown in Fig. 4 as an octet truss possessing non-compressible elongated elements having an open structure and repeating geodesic geometry.

The modules may be any geodesically delineated polyhedral structure or portion thereof. The module may be a fully geodesic polyhedron, such as a tetrahedron, or a more complicated omni-triangulated system, such as icosahedron (twenty sided polyhedron) a octahedron (eight sided polyhedron). Alternatively, the module may also contain non-triangular surface elements, such as square, pentagonal, hexagonal or octagonal facets. In other alternative embodiments, the module may be a more complicated polyhedron which itself can be further decomposed into simpler geodesic elements. For example, the module may comprise a half-dome, which itself may be comprised of tetrahedral, geodesic sub-modules. In certain embodiments, the members may form polyhedral modules with different shaped polygonal faces or only a subset of members mapping out geodesic lines.

The elongated members which comprise the modules are integral members of a single module, that is, they are not joined as separate elements but are formed as a unitary body. In embodiments where some kinematic properties are desired or where some flexibility at interstices is desired, it may be desired to provide elongated elements having differing cross-sectional areas near or at the interstices or vertices. Thus, in one embodiment, the modules are comprised of elongated elements which are "thicker" at the center and "narrower" at the vertices. Alternatively, the material properties of the scaffold material may be varied to provide increased compliance in the regions of the vertices, for example, by altering the cross-linking density of polymeric material.

The elongated elements may be non-compressible elements. Alternatively, the elongated elements may be extensible elements, that is, capable of extension or an increase in length in response to application of a tensile stress. Due to materials limitations, it is understood that such extensible properties will be experienced only over a limited range of motion. An extensible elongated element is expected to contract in length when compressed up to a certain point, at which point it will become non-compressible. Extensible members include but are not limited to linear (telescoping), curvilinear, helical, spring, sawtooth, crenulated or entanglement configurations. The scaffold material of the present invention may be comprised of all non-compressible elements, all extensible elements or a combination of the two.

Each of the modules is integrally connected to its neighboring modules so as to form the scaffold material. A scaffold forms a framework or internal skeleton upon which or into which additional materials may be introduced.

The elongated members, modules and hence the scaffold material itself may be prepared from any suitable material, dependent upon the desired application. For example, the scaffold may be prepared from non-erodible polymers such as, by way of example only, polyacrylates, epoxides, polyesters, polyurethanes, poly(methacrylate), polyimides, and polysiloxanes. Where flexibility is desired, such as where the structural members are extensible, the elongated members may be prepared using an elastomer. The materials selection of the elongated elements may be in part dictated by the method of manufacture and by the intended application, which are discussed hereinbelow.

The elongated members are of a dimension dictated by the intended application of the resultant scaffold material. However, the members will typically have a length in the range of about 1 x 10⁻⁹ m to about 1 x 10⁻¹ m, and more typically in the range of about 1 x 10⁻⁶ m to about 1 x 10⁻² m. The elongated members may be of a constant or varying thickness along their length, in particular where it is desired to impart localized flexibility to the structure. Typically, the cross-sectional diameter of the elongated element is in the range of about 1-1000 µm.

In many instances, the scaffold material is desirably prestressed in order to promote the self-stabilization of the tensegrity structure. Prestress may be introduced into the scaffold material in a variety of ways. By way of example only, the scaffold material may be prestressed by incorporating at least one non-compressible member and by contracting the other members of the material around the non-compressible member. Prestress may also be introduced by expansion or extension of a non-compressible member which is enclosed within the scaffold material. Alternatively, prestress may be imposed by incorporating a second material, such as a polymer, into the intrascaffold space of the material. In preferred embodiments, the second material is a polymer which is capable of swelling and which upon swelling imposes a stress on the scaffold material. Fig. 5 illustrates positioning of a polymer, preferably a swellable hydropolymer, into interstitial spaces so as to prestress the scaffold material while retaining an open pore structure which may be useful in some applications.

The scaffold material thus is an open network containing at least a portion of geodesic elements which may be integrally connected over an extended dimension, thereby forming sheets or other shapes as desired. The actual shape of the scaffold material and selection of the module geometries and dimensions may be selected to meet the needs of the intended application.

In one embodiment of the invention, the scaffold material may be used for filtration. A rigid octet truss such as shown in Fig. 4 is an example of a scaffold material suited for high flowthrough applications such as air masks, water purification systems, filtration, catalytic converters for removal of hydrocarbons and other detoxification networks. The open network may be impregnated with materials selected for their ability to remove particulants or chemicals from a medium. Typical impregants include, but are in no way limited to, hydrogels, charcoal particles, liposomes, lipid foams, detoxifying enzymes or catalysts, affinity binding ligands, antibodies and optical fibers. The bioactive hydrogels contemplated for use in the invention will significantly restrict passage of air, moisture, and heat, if applied as a solid layer. Thus, it will be necessary to construct these light-weight support scaffolds for the hydrogels that maintain open passages for air exchange, in addition to providing high tensile strength.

In another embodiment of the invention, the scaffold material may be incorporated into a pattern for shell investment casting. In this embodiment, a pattern is contructed which has a thin, solid outer surface, and an inner scaffold structure which supports the outer surface. The outer surface may be formed using the same process which is used to form the scaffold, or may be added after forming the scaffold, for example by wrapping a flexible material around a shaped scaffold.

The tensegrity-based structure of the scaffold allows the pattern to be made sufficiently strong to endure the casting process with a minimum of pattern material, and it optimizes porosity. Once formed, the surface of the pattern is coated with a hardenable material to form a shell coating. In one embodiment, the hardenable material may be a ceramic slurry which is cured to form a ceramic mold. The scaffold material is then eliminated by a method such as flash firing, leaving behind a shell suitable for casting metal or polymer pans in the shape of the original pattern. Techniques of forming a shell mold from a pattern for subsequent casting are well-known in the art, and are described in "Investment Casting," *Encyc. of Mat. Sci. & Eng*. **3**:2398-2402 (1986) and Stereolithography and other RP&M Technologies, Society of Manufacturing Engineers 183-185 (1996). An advantage of incorporating the types of scaffold material described herein into an investment casting pattern is that the amount of material necessary to support the shell mold is reduced compared to conventional patterns, reducing the amount of ash which is generated in firing. Further, these scaffolds contract more efficiently than conventionally used porous plastic patterns, and thus minimize expansive cracking of ceramic molds.

In a related embodiment, a similar pattern may be used to make a mold for sintering. In this embodiment, the pattern is used to produce a shell by forming a hardenable material around the pattern as in the previous embodiment. Once the pattern has been removed as described above, the shell can be filled with a powder and subjected to high temperature and/or high pressure to sinter the powder to produce a solid article, according to techniques well-known in the art. The shell may be removed after sintering or may form a part of the final article. Descriptions of sintering fundamentals can be found in "Sintering of Ceramics," *Encyc. of Mat. Sci. & Eng*. **6**:4455-4456 (1986) and "Physical Fundamentals of Consolidation," *Metals Handbook*, 9th ed., **7**:308-321.

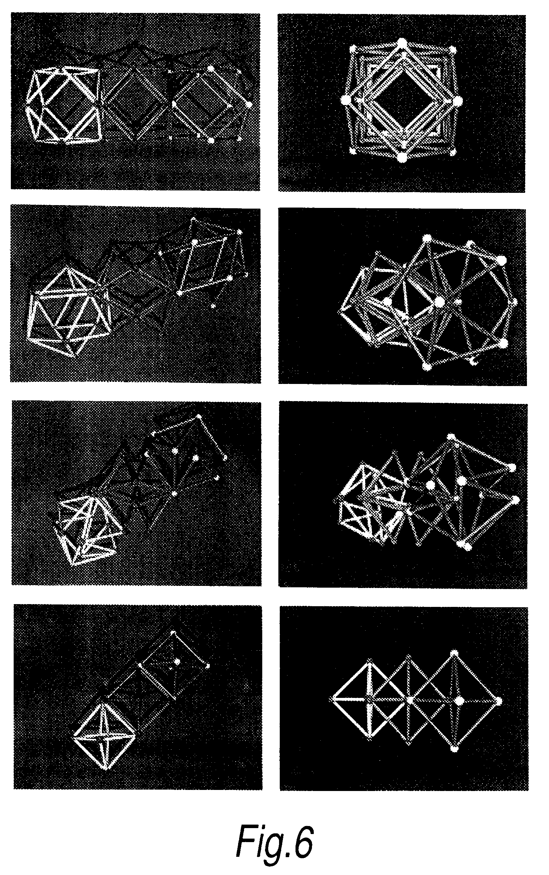

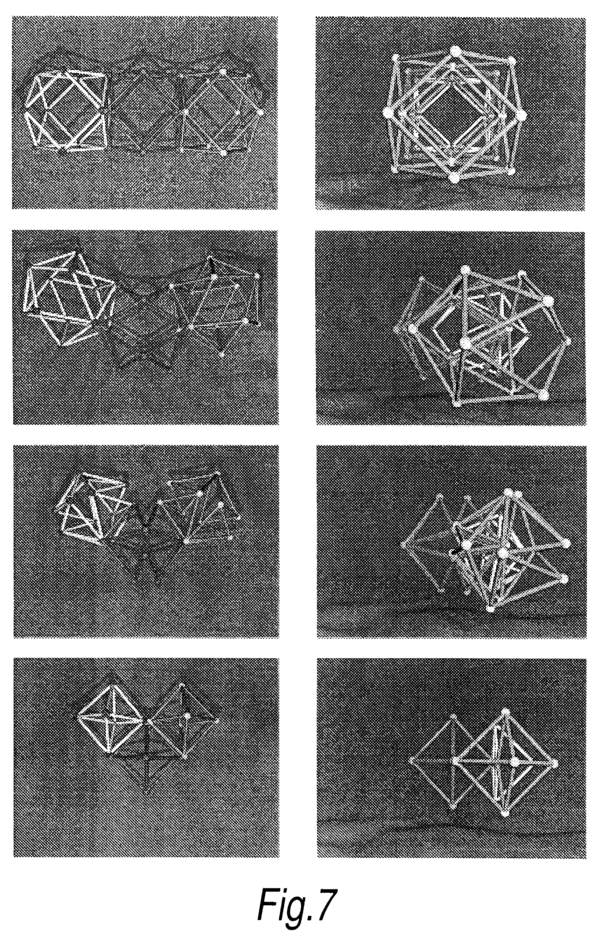

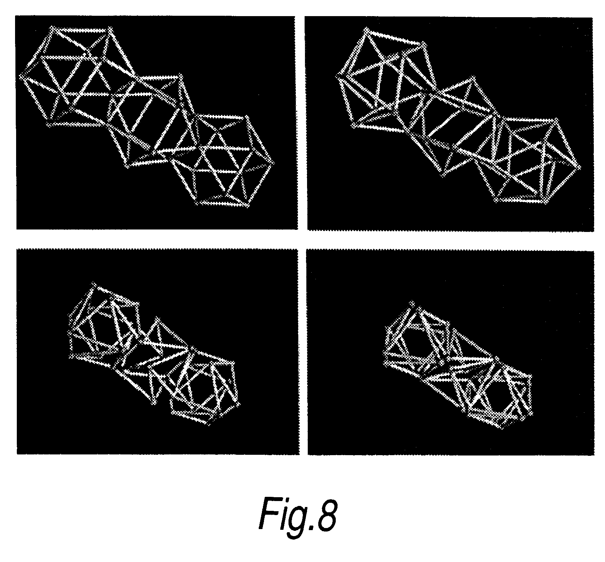

In another embodiment of the invention, the scaffold material may be prepared in long lengths as a textile. A scaffold material incorporating cuboctahedron modules integrally connected along one edge or face, as shown in Figs. 6-8, may provide a material which strengthens when compressed and thus may be useful against high force impacts, such as in bullet proof vests or umpire vests. In the embodiment illustrated in Fig. 6, an array of cuboctrahedron modules is capable of rearrangement into an array of octahedral modules. In another embodiment, a scaffold material incorporating cuboctahedron modules integrally connected along a single edge, different than that of Fig. 6, is capable of rearrangement into a "dog-leg" geometry and is particularly well-suited for textiles which may need to compact anisotropically around curved or angular surfaces (Fig. 7). Fig. 8 illustrates yet another embodiment, in which cuboctahedron modules rearrange into yet another configuration of octahedral modules. All three materials would maintain high flexibility and porosity in their open configuration, while exhibiting high rigidity, strength, and a restriction to flow-through when compacted and thus, may be useful for construction of filtration systems that automatically shut off and valve themselves at high pressure.

In another embodiment, the textile may be adapted for use as battledress overgarments (BDO) for protection against biological or chemical toxins. Conventional BDOs rely on use of activated charcoal particles or other means to absorb air-borne chemical toxins. They offer little or no protection against biological toxins or pathogens. To be effective against biological threats at high concentrations, the scaffold material of the present invention may be fabricated to exhibit high porosity and tortuosity to permit pathogen entry and air passage while physically restricting pathogen penetration. To ensure efficient biothreat removal, the fabric desirably exhibits a high surface area to volume ratio and contains molecular ligands and enzymes that bind, sequester, and destroy toxins and pathogens with high efficiency. To facilitate binding interactions and support enzymatic activities, the material desirably retains a significant amount of water and thus is organized, at least in part, as a "hydrogel." The water phase also may enhance removal of biological and chemical agents contained within air-borne water droplets as the air passes through the high surface area of the lattice and thereby, further increase the efficiency of pathogen capture. In one embodiment of the present invention, the scaffold material is desirably coated with or impregnated with a hydrogel.

Suitable hydrogels include, but are not limited to, poly(2-hydroxyethyl methacrylate-co-methyl methacrylate), copolymers of methacrylic acid (MAA), acrylic acid (AA), and/or glycidyl methacrylate (GMA), unsaturated linear polyesters, and poly(ethylene glycol). In the preparation of poly(2-hydroxyethyl methacrylate-co-methyl methacrylate), the monomers, 2-hydroxy ethyl methacrylate (HEMA) and methyl methacrylate (MMA), are liquid and can be mixed with a crosslinking agent (ethyleneglycol dimethacrylate, EGDMA) and an initiator to polymerize and form a hydrogel. The relative amount of 2-hydroxyethyl methacrylate confers hydrophilicity and also pliability whereas the methyl methacrylate confers hydrophobicity and mechanical rigidity to the hydrogel. The engineering of the HEMA/MMA copolymer ratio and the EDGMA crosslinking ratio allows one to synthesize hydrogels with tailored perinselective and mechanical properties.

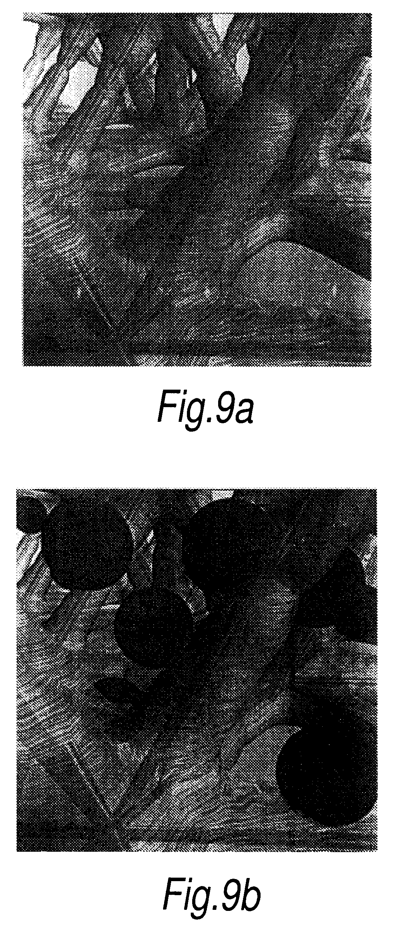

The tensile strengths and moduli of the materials may be determined by an axial/torsional test system. Water binding capacities can be measured in dynamic and equilibrium swelling studies. Porosity and pore size of dry specimens can be measured by mercury intrusion porosimetry. The porosity and pore size of wet specimens can be estimated morphometrically using environmental scanning electron microscopy. Fig. 9a illustrates a scaffold material which has been coated with a hydrogel.

It also should be possible to provide additional protective activity against biothreat agents by incorporating enzymes (e.g., proteases to breakdown toxins; enzymes that generate free radicals in the gel). In addition, enzymes that bind, inactivate, and/or destroy chemical toxins, such as organophosphorus nerve gases (e.g., acetylcholinesterase, organophosphorus acid anhydrolase, phosphotriesterase), also could be incorporated within the gel to provide additional protection against these chemical warfare agents. By way of example only, acetylcholinesterase enzyme may be conjugated to a hydrogel using carbonyl-diimidazole and the activity of the immobilized enzyme analyzed in vitro using conventional biochemical techniques. The efficiency of protein binding also can be assessed by radiolabeling or surface analysis (X-ray photoelectron spectroscopy).

Biological toxins (e.g., ricin toxin, botulinum toxin, Vibrio cholerae neuraminidase) and pathogenic organisms (e.g., bacteria, viruses, protozoa) adhere to living cells and enter the body by binding to specific ligands on the cell surface, including simple sugars (e.g., galactose), complex carbohydrates (e.g.. heparan sulfate), and membrane phospholipids (e.g., phosphatidyl-inositol). Monoclonal antibodies also have been generated which bind to specific biothreat agents with high affinity (e.g., ricin toxin). These pathogenic organisms and toxins may be bound and sequestered by conjugating these specific ligands to the backbone of a hydrogel which has been impregnated into the scaffold material.

A technique has been developed to coat poly(HEMA) hydrogels onto porous scaffolds, including geodesic scaffold cassettes fabricated using stereolithography. The procedure consists of an initial pre-coating process using HEMA/alcohol solution, followed by polymerization of HEMA and the crosslinking agent EGDMA in 0.7 M NaCl using a redox initiating system. A uniform hydrogel coating can be obtained using this method. The thickness of the hydrogel coating is controlled by the concentration of monomer and initiator, as well as coating time.

The scaffold material of the present invention may be prepared using well known polymer synthesis and microfabrication techniques including, but not limited to, stereolithography, three dimensional microprinting, microscale patterning and micromolding techniques. These fabrication techniques may be facilitated by the use of mathematical models for cell and cytoskeletal mechanics based on tensegrity. These mathematical principles are described in "A Microstructural Approach to Cytoskeletal Mechanics based on Tensegrity" *J. Theor. Biol.* **181**:125 (1996), which is found in Appendix I. The two parameters that determine the mechanical stability of tensegrity structures are prestress and architecture. Prestress determines the initial stiffness of the structure and ensures that it will respond immediately when external force is applied. Architecture describes the number of different building elements and how they distribute forces in space. This geometric feature determines how the different structural elements rearrange and thus, how the entire structure stiffens in response to stress. This mathematical treatment provides an entirely new quantitative method for analyzing the mechanical properties of any natural system that exhibits linear stiffening behavior, regardless of size.

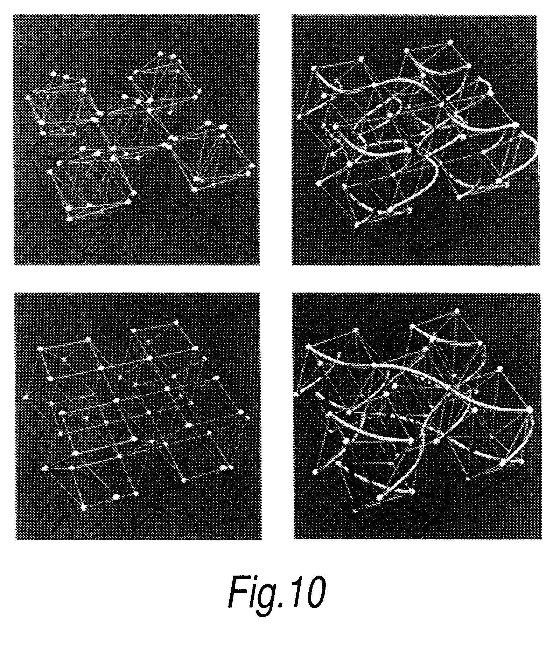

Tensegrity structures can be complex, however, and equations and analytical solutions alone may not give the full picture of how these structural networks react to external forces. For this reason, computer modeling and animation techniques may be used to simulate and explore dynamic geometric transformations within three dimensional (3D) tensegrity lattices as an aid to the design of the scaffold materials of the invention. This CAD capability makes possible the designing of synthetic networks with desired mechanical, structural, and geometric features. For example, the computer-generated time sequences depicted in Figs. 6-8 show highly flexible and porous lattices containing large central pores undergoing progressive geometric transformations (left and right columns are top and side views of the same structure, respectively). Note that the loose, open lattices progressively fold in on themselves, close their central pores, and mechanically stiffen (reorganizes into stable octagonal and tetrahedral forms), without breaking any structural connections. A synthetic polymer lattice fabricated with the same geometric features offers a similar ability to vary network mechanics, organization, and porosity, without loss of structural integrity. Furthermore, the mechanical strength of these flexible network can be greatly increased, by incorporating larger, less flexible struts (e.g., long carbon fibers) within the framework as shown in Figure 10. A similar approach may be used to generate anisotropic behavior in different regions of biomimetic textile fabrics (e.g., over a knee joint).

In recent years, manufacturers have been foregoing blueprints and drawing boards in favor of workstations and CAD/CAM software. Using commercial software, engineers are able to convert CAD drawings into interactive, high-resolution 3D models. They can rotate parts and check them for form, fit, and function without ever holding a part in their hands. Using linked CAD/CAM systems they can directly translate design into product. At the same time, work with materials manufactured for the aerospace and automobile industries, and particularly with composite materials, has revealed that the microstructure of these materials (e.g., dimension and orientation of fibers) largely determines their mechanical properties. In these materials, most features of the microstructure are beyond the control of the design/manufacturing process. These small scale structural features are created essentially as an artifact as the material is being formed into its final shape using conventional processes such as injection or compression molding.

Combination of CAD technology with CAM software allows fabrication of scaffold materials with defined geodesic and tensegrity-based microstructures which have been identified as desirable. Specifically, CAD capability may be combined with CAM technology based on stereolithography. In stereolithography, a liquid polymer resin is selectively polymerized (solidified) by a laser beam chat is under the control of a computer to construct a polymeric material with 3D microstructural features that precisely match those specified using CAD. The construction process involves fabrication of sequential chin cross section layers (analogous to tomographic sections), one being polymerized atop the other, until the entire 3D material is completed. Using this approach, 3D porous polymer networks can be fabricated with any microstructure that can be created using CAD. Although epoxy-based resins are most commonly used in this technique, in theory, any chemical that may be polymerized using a UV-sensitive initiator may be utilized. An octet truss configuration, composed of closedly packed octahedra and tetrahedra, has been produced using both epoxy and acrylate resins using stereolithography.

This CAD capability allows the design and fabrication of materials with capabilities never previously considered. By way of example, the same approach that leads to development of mechanically strong and light polymer skins for pathogen neutralization may be adapted to develop bioerodible polymer scaffolds for tissue engineering or biomimetic erythrocytes (bioerodible nanosponges) that can be injected intravascularly to soak up and neutralize pathogens in vivo. Possible materials for such applications include, but are not limited to, polyglycolic acid, polylactic acid, polyimide, polyamide, polyester, protein, carbohydrate, nucleic acid, and lipid.

Other well-known microfabrication techniques may be used in the preparation of the scaffold material. By way of example only, stereolithography techniques, three dimensional microprinting, three dimensional laser-based drilling or etching techniques, micromolding, and self-assembly methods may be used in the preparation of the material of the invention. The interested reader is directed to Science and Technology of Microfabrication R.E. Howard, E. L. Hu, S. Namba, S. Pang (Eds.) Materials Research Society Symposia Vol. 76 (1987), for further information on microfabrication.

A typical scaffold material that could be used for a protective air mask filtration device has been prepared as described in the following example.

A CAD application was used to design a three dimensional network. Several commercially available programs, including ProEngineer (Parametrics Technology Corporation) and Ideas (Structural Dynamics Research Corporation), have been found suitable for designing the network. The network consisted of an octet truss such as shown in Fig. 4, with member lengths of about 800 µm and member transverse cross-sectional diameters of about 150 µm. All struts were oriented at 60 degree angles relative to neighboring members, resulting in the formation of a continuous array of closely packed tetrahedron modules with octahedral intermodule spaces or what is known as an "octet truss." The scaffold material has been fabricated using both epoxy and acrylate resins, using CAM in combination with a 3D Systems SLA-250 Stereolithography apparatus (3D Systems, Valencia, CA). This apparatus, modified to include a TEM 00 laser, can produce up to 23" cube of polymer composed of a unitary epoxy or acrylate resin with the finest line features (elongated members) of about 70 µm² in cross-sectional area.

Once formed, the scaffold was immersed in liquid hydrogel resin containing HEMA and EGDMA, then purged with air to remove most free resin. The residual resin adherent to the surface of the scaffold material was polymerized in 0.7 M NaCl using a redox initiating system to form a hydrogel coating as visualized in Fig. 9a. This hydrogel could in turn be chemically conjugated to biactive molecules such as carbohydrates containing galactose dimers to remove ricin toxins, antibodies against different pathogenic organisms, and enzymes, such as acetylcholinesterase, that may deactivate nerve gases. The scaffold spaces could also be impregnated with charcoal microparticles (Fig. 9b) to remove air-borne chemical toxins as well as liposomes or lipid foams to remove lipophilic agents and organisms.

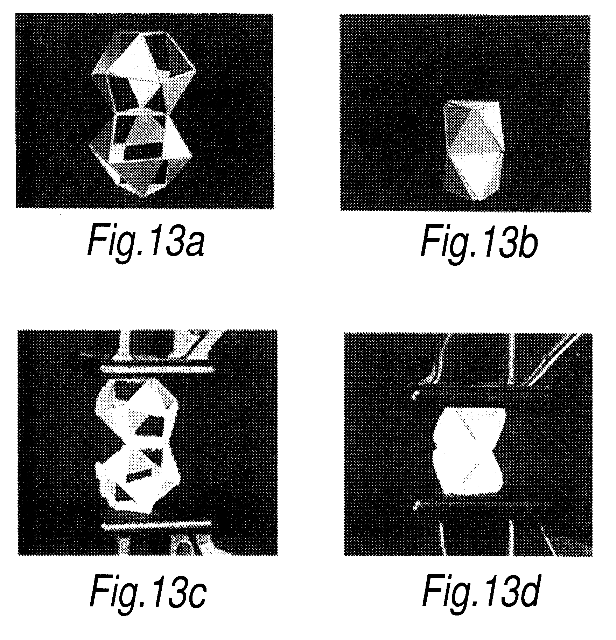

A prototypical flexible array of cuboctahedrons has also been produced using Dupont SOMOS 2100 polymer. The prototype, illustrated in Figure 13c and 13d, consisted of two linked cuboctahedra, but larger arrays could be made by the same techniques.

The flexible array was made using the same CAD/CAM systems used to manufacture the octet truss described above. The prototype constructed had a height of 33mm in the fully expanded configuration, and a height of 17mm in the contracted configuration shown in Figure 13d. The prototype was found to deform relatively easily as the cuboctahedrons collapsed into octahedrons, and then to stiffen considerably. This behavior was in accord with computer models of this configuration. Figures 13a and 13b depict computer models of the prototype in an expanded and a contracted configuration. A large array made using the principles of this prototype would maintain high flexibility and porosity in the open configuration, while exhibiting high rigidity, strength, and a restriction to flow-through when compacted and thus, could be useful for construction of filtration systems that automatically shut off and valve themselves at high pressure.

Another example of a scaffold material which can be made using CAD/CAM is a cuboctahedron scaffold in which vertices are narrowed so as to permit greater flexibility in the material. By way of example, each member is about 1 mm in length, about 275 µm in cross-section diameter at its center and about 250 µm in diameter at each vertex. Such a scaffold would behave much like the flexible prototype described above, except that flow-through would still be possible even in the compacted configuration, since the two-dimensional triangle elements would be replaced by one-dimensional struts along the edges of the triangles.

Other embodiments of the invention will be apparent to those skilled in the art from a consideration of the specification or practice of the invention disclosed herein and, in particular they may include the features listed in the following enumerated paragraphs ("paras").
1. A scaffold material, comprising:
   a predetermined arrangement of integrally connected modules, each said module comprised of a plurality of integrally connected elongated members forming at least a portion of a polyhedron, the members arranged such that at least a portion of said members form geodesic or tensegrity elements.
2. The material of para 1, wherein said modules are capable of rearranging to form a fully geodesic or tensegrity structure.
3. The material of para 1, wherein all members within all said modules form geodesic or tensegrity elements.
4. The material of para 1, wherein said elongated members are substantially non-compressible.
5. The material of para 1 in which a first portion of said members are substantially non-compressible and a second portion of said members are extensible.
6. The material of para 1 or 2, wherein said modules comprise a subset of members oriented as non-geodesic four-sided polygonal elements.
7. The material of para 1 or 3, wherein said polyhedral module comprises eight triangular elements integrally connected at their common vertices.
8. A scaffold material, comprising:
   a predetermined arrangement of integrally connected modules, each said module comprised of a plurality of integrally connected, elongated, extensible members forming a least a portion of a polyhedron, the members arranged such that all said members form geodesic elements.
9. The material of para 8, wherein said extensible member is selected from the group consisting of linear, curvilinear, helical, spring, sawtooth form, crenulated, and entanglement elements.
10. The material of para 1, 2, 3, or 8, wherein said module is selected from a set comprising a sphere, a portion of a sphere, an icosahedron, an octahedron, a dodecahedron, tetrahedron, and truncated or stellated forms of these geodesic polyhedra.
11. The material of para 1, 2, 3, or 8, wherein said arrangement comprises a plurality of said modules integrally connected by sharing one or more common vertices, edges, faces or intersections.
12. The scaffold material of para 1, 2, 3 or 8, wherein said members have an elongated dimension in the range of about 1 x 10⁻⁹ to about 1 x 10⁻¹ meter.
13. The scaffold material of para 1, 2, 3 or 8, wherein the arrangement of the geodesic or tensegrity elements results in a structure having intermember pores in the range of about 1 x 10⁻⁹ to about 1 x 10⁻¹ meter.
14. The scaffold material of para 1, 2, 3, or 8, wherein at least one module is prestressed.
15. The scaffold of para 14, wherein said prestress is imposed by a polymer located within the at least one module.
16. The scaffold material of para 15, wherein said polymer is a hydrogel capable of swelling when placed in contact with an aqueous medium.
17. The scaffold material of para 16, wherein said hydrogel polymer is composed of poly(2-hydroxyethyl methacrylate-co-methyl methacrylate), unsaturated linear polyesters and poly(ethylene glycol), copolymers of methacrylic acid, acrylic acid, and/or glycidal methacrylate.
18. The material of para 1, 2, 3 or 8, further comprising a hydrogel polymer coated onto at least a portion of the elongated members.
19. The scaffold material of para 18, wherein said hydrogel polymer is composed of poly(2-hydroxyethyl methacrylate-co-methyl methacrylate), unsaturated linear polyesters and poly(ethylene glycol), copolymers of methacrylic acid, acrylic acid, and/or glycidal methacrylate.
20. The scaffold material of para 18, wherein said polymer is derivatized with biologically or chemically active molecules.
21. The scaffold material of para 15, wherein said polymer is capable of expansion upon exposure to heat, change in pH, change in electrical charge or other environmental condition.
22. The scaffold of para 14, wherein said prestress is imposed by incorporating at least one non-compressible member.
23. The scaffold of para 22, wherein said prestress is imposed by contraction of the scaffold material around said at least one non-compressible member.
24. The scaffold of para 22, wherein said prestress is imposed by expansion of the at least one non-compressible member into contact with the scaffold material.
25. The scaffold material of para 1, 2, 3, or 8, wherein said members of said module define at least one intermember space and the at least one intermember space is filled with a solid material.
26. The scaffold material of para 25, wherein said solid material is an elastomer.
27. The scaffold material of para 26, wherein said elastomer is selected from the group consisting of unsaturated linear polyesters and poly(ethylene glycol), polyurethane, and polydimethylsiloxane.
28. The scaffold material of para 1, 2, 3, or 8, wherein the structural members are composed of non-erodible polymers.
29. The scaffold material of para 28, wherein said polymer is selected from the group consisting of polyacrylates, polyepoxides, polyesters, polyurethanes, poly(methacrylic acid), poly(acrylic acid), polyimides, and polysiloxanes.
30. The scaffold material of para 1, 2, 3, or 8, wherein the structural members are composed of erodible polymers.
31. The scaffold material of para 30, wherein the structural members are selected from the group consisting of polyglycolic acid, polylactic acid, polyiimide, polyamide, polyester, protein, carbohydrate, nucleic acid, and lipid.
32. The scaffold material of para 1, 2, 3, or 8, wherein the said material is fabricated using computer aided manufacturing techniques.
33. The scaffold material of para 32, wherein said material is fabricated using at least one of stereolithography, micromolding, three dimensional microprinting, three dimensional laser-based drilling or etching, and self-assembly techniques.
34. The scaffold material of para 1, 2, 3, or 8, wherein a portion of the modules is derivatized with biologically or chemically active molecules.
35. The scaffold material of para 1, 2, 3, or 8, wherein the surface of said material is coated with a polymer.
36. The scaffold material of para 35, wherein the said coating polymer is derivatized with biologically or chemically active processing molecules.
37. The scaffold material of para 1, 2, 3, or 8, wherein the scaffold forms the internal structure of a pattern for a manufactured article.
38. The scaffold of para 37, wherein the pattern is a pattern for investment casting.
39. The scaffold of para 37, wherein the manufactured article is to be formed by sintering.
40. A pattern for a manufactured article, comprising
   solid outer surfaces forming the shape of the article to be manufactured, and
   an internal scaffold material, comprising a predetermined arrangement of integrally connected modules, each said module comprised of a plurality of integrally connected elongated members forming at least a portion of a polyhedron, the members arranged such that at least a portion of said members form geodesic or tensegrity elements.
41. The pattern of para 40, wherein the article is to be manufactured by investment casting.
42. The pattern of para 40, wherein the article is to be manufactured by sintering.
43. A method of creating a mold for manufacturing, comprising:
   (a) providing a pattern in the shape of an article to be manufactured, comprising:
      (i) solid outer surfaces, and
      (ii) an internal scaffold material, comprising a predetermined arrangement of integrally connected modules, each said module comprised of a plurality of integrally connected elongated members forming at least a portion of a polyhedron, the members arranged such that at least a portion of said members form geodesic or tensegrity elements,
   (b) coating the pattern with a hardenable material,
   (c) transforming the hardenable material into a hard shell mold, and
   (d) removing the pattern from the shell without breaking the hard shell mold.
44. The method of para 43, wherein the mold is to be used for casting a metal, ceramic, glass, or polymer article.
45. A method of investment casting, comprising:
   (a) providing a pattern in the shape of an article to be formed by casting, comprising:
      (i) solid outer surfaces, and
      (ii) an internal scaffold material, comprising a predetermined arrangement of integrally connected modules, each said module comprised of a plurality of integrally connected elongated members forming at least a portion of a polyhedron, the members arranged such that at least a portion of said members form geodesic or tensegrity elements,
   (b) coating the pattern with a hardenable material,
   (c) transforming the hardenable material into a hard shell,
   (d) removing the pattern from the shell without breaking the shell, and
   (e) casting the article by filling the shell with liquid which is subsequently transformed into a solid.

## Claims

1. A scaffold material, comprising:
a predetermined arrangement of integrally connected modules, each said module comprised of a plurality of integrally connected elongated members forming at least a portion of a polyhedron, the members arranged such that at least a portion of said members form geodesic or tensegrity elements.

2. A scaffold material, comprising:
a predetermined arrangement of integrally connected modules, each said module comprised of a plurality of integrally connected, elongated, extensible members forming at least a portion of a polyhedron, the members arranged such that all said members form geodesic elements.

3. A pattern for a manufactured article, comprising:
solid outer surfaces forming the shape of the article to be manufactured, and
an internal scaffold material, comprising a predetermined arrangement of integrally connected modules, each said module comprised of a plurality of integrally connected elongated members forming at least a portion of a polyhedron, the members arranged such that at least a portion of said members form geodesic or tensegrity elements.

4. A method of creating a mold for manufacturing, comprising:
(a) providing a pattern in the shape of an article to be manufactured, comprising:
(i) solid outer surfaces, and
(ii) an internal scaffold material, comprising a predetermined arrangement of integrally connected modules, each said module comprised of a plurality of integrally connected elongated members forming at least a portion of a polyhedron, the members arranged such that at least a portion of said members form geodesic or tensegrity elements,
(b) coating the pattern with a hardenable material,
(c) transforming the hardenable material into a hard shell mold, and
(d) removing the pattern from the shell without breaking the hard shell mold.

5. A method of investment casting, comprising:
(a) providing a pattern in the shape of an article to be formed by casting, comprising:
(i) solid outer surfaces, and
(ii) an internal scaffold material, comprising a predetermined arrangement of integrally connected modules, each said module comprised of a plurality of integrally connected elongated members forming at least a portion of a polyhedron, the members arranged such that at least a portion of said members form geodesic or tensegrity elements,
(b) coating the pattern with a hardenable material,
(c) transforming the hardenable material into a hard shell,
(d) removing the pattern from the shell without breaking the shell, and
(e) casting the article by filling the shell with liquid which is subsequently transformed into a solid.
